# FASCICULE DE BREVET EUROPEEN

(11) **EP 3 082 630 B1**
(45) Date de publication et mention de la délivrance du brevet: **28.03.2018**
(21) Numéro de dépôt: 14830828.1
(22) Date de dépôt: 18.12.2014
(51) Int. Cl.: A61B 17/70

(54) **DISPOSITIF DE FIXATION VERTEBRALE A DOUBLE ACCROCHE, SYSTEME DE BLOCAGE D'UNE BOUCLE AVEC UN TEL DISPOSITIF**
WIRBELFIXIERVORRICHTUNG MIT DOPPELTER BEFESTIGUNG, SYSTEM ZUR BLOCKIERUNG EINER SCHLEIFE MIT SOLCH EINER VORRICHTUNG
VERTEBRAL FIXATION DEVICE WITH DOUBLE FASTENING, SYSTEM FOR BLOCKING A LOOP WITH SUCH A DEVICE

(30) Priorité: 19.12.2013 FR 1363093
(43) Date de publication de la demande: 26.10.2016
(73) Titulaire: Implanet, 33650 Martillac (FR)
(72) Inventeur: LE COUËDIC, Régis, F-33000 Bordeaux (FR); BACCELLI, Christian, F-33650 Saucats (FR)
(74) Mandataire: Benech, Frédéric
(86) Numéro de dépôt international: PCT/FR2014/053429
(87) Numéro de publication internationale: WO 2015/092300

(56) Documents cités:
- FR-A1- 2 954 905
- US-A1- 2013 018 428
- US-A1- 2013 072 983
- US-A1- 2013 150 892
- US-B2- 8 465 527

## Description

La présente invention concerne un dispositif de fixation vertébrale pour le maintien d'une vertèbre rachidienne sur une tige, du type comportant un corps de fixation sur la tige, une bande souple de liaison de la vertèbre avec le corps de fixation et des moyens de blocage réglable de la bande souple sur le corps de fixation.

Elle concerne également un système d'ancrage mettant en oeuvre un tel dispositif.

Elle trouve une application particulièrement importante, bien que non exclusive, dans le domaine du redressement de la colonne vertébrale d'un patient présentant une courbure anormale et plus particulièrement en cas de réduction d'une grande scoliose de la colonne vertébrale.

On sait que, en cas de scoliose, les vertèbres n'étant pas alignées correctement les unes par rapport aux autres par rapport à l'axe vertébral, elles présentent des inclinaisons entre elles.

Afin de redresser l'ensemble, il est connu de remettre à une distance sensiblement équivalente les bords des vertèbres de part et d'autre de la colonne vertébrale, par le biais de tiges reliant entre elles, soit des vis, que l'on insère dans les vertèbres elles-mêmes, soit des crochets, que l'on introduit le long du canal rachidien.

De tels dispositifs présentent cependant des inconvénients. En particulier, les systèmes à vis nécessitent des vertèbres en bon état général, tandis que les systèmes à crochets sont très délicats à manipuler en raison de la proximité de la moelle épinière et présentent donc des risques non négligeables pour le patient.

De tels systèmes sont de plus difficiles à placer par le chirurgien, le crochet ayant tendance à se décrocher pendant l'opération, tant que le reste de la tige n'est pas bloquée.

En particulier, dans le cas de grandes scolioses réduites et complexes, les contraintes mécaniques peuvent s'avérer extrêmement importantes et risquent plus sûrement encore de faire se désolidariser les implants des vertèbres sur lesquelles ils sont fixés.

On connaît des solutions de fixation très résistantes qui font appel à des implants à double crochets, dit pince-crochets, qui permettent d'enserrer la lame de la vertèbre thoracique entre deux crochets, ce qui autorise une fixation pérenne sur la vertèbre avec la tige d'union longitudinale de montage.

De telles fixations nécessitent de prévoir une connexion des deux côtés, gauche et droit de la vertèbre, entrainant une réalisation délicate à partir de deux crochets, qui se font face.

En particulier, le rapprochement de la tige et des crochets est problématique.

On connaît (US 8,465,527), un système à crochet ajustable sur une première partie osseuse comportant un lien flexible agencé pour coopérer avec une seconde partie osseuse permettant un rapprochement progressif et sécurisé.

Toutefois, cette double fixation, qui ne concerne pas à proprement parlé une fixation sur une tige, ne permet pas d'éviter de casser le lien flexible lors de fortes sollicitations.

En effet, dans un tel système, le lien flexible et le crochet fixent deux parties distinctes de l'anatomie rachidienne de sorte que la contrainte est reprise en totalité par le lien flexible qui risque la rupture.

On connaît également (US 2013/0150892) un système de stabilisation de vertèbre comprenant un corps muni d'un crochet et d'une bande flexible en vis à vis du crochet. Si un tel système vient sécuriser la tenue de la vertèbre en venant comprimer la bande sur la vertèbre par le crochet on comprend que la bande fonctionne toujours en traction. Ici encore la contrainte est donc reprise par le lien flexible (qui empêche le crochet de sortir de la vertèbre). Elle risque de ce fait toujours de se desserrer et/ou de se rompre avec le temps. FR 2 954 905 décrit un dispositif de fixation vertébrale pour maintien d'une vertèbre rachidienne sur une tige, comportant un corps de fixation sur la tige, une bande souple de liaison de ladite vertèbre avec le corps de fixation et des moyens de blocage réglable de la bande souple sur le corps de fixation. Le corps de fixation est formé d'une seule pièce de section transversale en forme de U, de passage de la tige entre la paroi de fond du U et les moyens de blocage réglable. Les moyens de blocage réglable sont formés par une pièce de liaison reliant les extrémités en vis à vis des deux branches du U, agencée pour comprimer la tige contre la paroi. Les branches comprennent chacune un évidement en vis à vis situé du coté du fond du U, de passage entre ledit fond et la tige des deux extrémités de la bande propre à former une boucle de fixation sur une vertèbre. La présente invention vise à pallier les inconvénients de l'art antérieur.

La présente invention est définie par la revendication indépendante 1, tandis que des modes de réalisation préférés sont exposés dans les revendications dépendantes.

Pour ce faire elle vise à fournir un dispositif et un système de fixation vertébrale répondant mieux que ceux antérieurement connus aux exigences de la pratique, notamment en ce qu'elle va permettre de stabiliser l'implant tant qu'il n'est pas connecté à la tige, qu'elle permet d'utiliser la tresse comme moyen de guidage du crochet préalablement clippé sur la tige d'union afin d'amener le couple crochet/tige au contact de la lame vertébrale, qu'elle autorise également la prise en charge des sollicitations mécaniques dépassant les capacités de la tresse polymère qui est utilisée, qu'elle permet une pose simplifiée de l'ancrage sur
le même montage et ainsi une réduction des temps opératoires et une approche progressive du crochet en utilisant une capacité de réduction de la tresse.

Le dispositif selon l'invention est aisément manipulable, solide et économique, permettant à tout moment, depuis la pose du dispositif de fixation et pendant toute la durée d'utilisation du système, d'assurer une fixation robuste et de garder les éléments fixés entre eux (vertèbre et dispositif de fixation) à une distance fixe les uns par rapport aux autres.

Le dispositif va également selon l'invention assurer un lien permanent réglable entre la vertèbre et les éléments de liaison crochet/tige en assurant un rapprochement progressif du crochet et de la tige vers la vertèbre.

L'invention évite ainsi tout risque de rupture du lien flexible, même en cas de sollicitations mécaniques très importantes.

Dans ce but, l'invention propose notamment un dispositif de fixation vertébrale pour maintien d'une vertèbre rachidienne sur une tige, comportant un corps de fixation sur la tige, une bande souple de liaison d'une partie de ladite vertèbre avec le corps de fixation et des moyens de blocage du corps sur la tige et de la bande souple sur le corps de fixation via la tige. Le corps de fixation comprend de plus un crochet d'ancrage rigide, solidaire du corps de fixation, apte à venir se fixer sur la partie de vertèbre à proximité immédiate de la bande souple en position sur ladite partie de vertèbre et des moyens de passage de la bande entièrement à distance du crochet pour former en partie les moyens de blocage du corps sur la tige sans interférer avec le crochet.

Plus avantageusement, l'invention propose un dispositif de fixation vertébrale pour maintien d'une vertèbre rachidienne sur une tige selon la revendication 1. Par proximité immédiate on entend une distance inférieure à de l'ordre de dix millimètres, par exemple inférieure à 5 mm ou en contact avec.

Avantageusement le crochet est situé de l'autre côté du plan parallèle au passage de la bande, défini par l'axe de la tige, par rapport au passage formé par les évidements.

Par crochet parallèle à la tige, on entend notamment un crochet dont la section longitudinale est dans un plan parallèle au plan dans lequel s'inscrit la tige perpendiculaire au plan de la section en U.

On notera que, relativement au crochet ou moyen d'ancrage, l'expression « apte à venir se fixer » doit être entendue comme agencée pour former une fixation mécanique, ne nécessitant aucune pièce additionnelle telle qu'une vis ou un écrou. Des particularités de cette fixation du moyen d'ancrage seront données dans la suite.

Ainsi, en contradiction avec les enseignements de l'art antérieur qui limitent à un seul moyen de fixation la liaison avec une vertèbre, l'invention propose de disposer de deux moyens de fixation simultanés, l'un des moyens étant insérable de façon aisément décrochable sur la vertèbre en l'absence de tension, (le crochet d'ancrage) et l'autre non car nécessitant d'échapper la totalité d'une extrémité de la bande (la bande souple enserrant la vertèbre).

De plus, ces deux moyens de fixation ne sont pas destinés à venir fixer deux parties distinctes et
souvent distantes d'éléments osseux qu'il convient de rapprocher, ainsi que cela est le cas dans l'art antérieur, mais au contraire ils viennent fixer une tige à une même partie de l'anatomie rachidienne, les deux moyens de fixation étant placés à proximité l'un de l'autre.

On entend par l'expression « partie de l'anatomie rachidienne » une région de la vertèbre formant une section s'étendant sensiblement linéairement à savoir une lame vertébrale ou un pédicule vertébral. On comprend que l'important réside dans le fait que les deux fixations, la bande souple et le crochet d'ancrage, sont agencés pour être fixées sur cette même partie d'anatomie rachidienne.

Avantageusement la bande souple de liaison s'étend au repos dans un plan d'extension sensiblement perpendiculaire à l'axe de la tige et étant apte à venir se fixer sur une partie de l'anatomie rachidienne (c'est-à-dire en l'espèce la vertèbre) en enserrant totalement cette dernière lorsqu'en position de travail.

On entend par le terme « sensiblement » dans l'expression relative au plan d'extension de « la bande souple s'étendant au repos dans un plan d'extension sensiblement perpendiculairement à l'axe de la tige » le fait que l'axe linéaire X'X de la tige coupe le plan P formé par la bande souple au repos selon un angle à 90° ± 15°, préférentiellement selon un angle à 90° ± 5°.

L'expression « au repos », relativement à la bande souple de fixation, signifie l'état de cette dernière lorsque sa boucle est libre, c'est-à-dire qu'elle n'enserre pas une partie de l'anatomie rachidienne (vertèbre). Par opposition, la position de travail de la bande souple est obtenue lorsque cette dernière enserre la partie de l'anatomie rachidienne.

Avec l'invention et grâce en particulier au positionnement respectif de la bande souple et du crochet d'ancrage, il est dorénavant possible, notamment :
- de permettre que le dispositif de fixation soit manipulé bien plus aisément par un opérateur. Ainsi, à titre d'exemple, la bande souple, lorsqu'elle est en prise sur ou avec la partie de l'anatomie rachidienne, permet de stabiliser le moyen d'ancrage tant que le corps de fixation n'est pas connecté à la tige ;
- de prendre en charge les sollicitations mécaniques dépassant à terme les capacités de résistance de la bande souple, sans créer de risques de rupture intempestifs, en particulier qui seraient dus à la contrainte en traction exercée sur la bande, mais également du fait des torsions ou autres forces parasites ;
- de simplifier la pose du système de fixation et ainsi de réduire le temps opératoire ;
- de permettre l'approche progressive du moyen d'ancrage en utilisant les capacités de réduction de longueur de la bande souple.

Dans des modes de réalisation avantageux, on a de plus et/ou par ailleurs recours à l'une et/ou à l'autre des dispositions suivantes :
- le crochet d'ancrage présente une section transversale sensiblement en forme de L (en d'autres termes, et considérant également que la branche inférieure du corps de fixation d'où s'étend le moyen d'ancrage est à prendre en compte, une forme sensiblement en U) de manière à venir en prise et/ou agencée pour s'insérer sur la partie de la vertèbre concernée ;
- le crochet d'ancrage présente une section de forme au moins en partie complémentaire à la partie de l'anatomie rachidienne sur laquelle il est destiné à venir en prise afin d'assurer une fixation amovible par complémentarité de formes. Dans ce cadre, on comprend que sa forme ou sa géométrie présente une importance particulière puisque sa fonction, à savoir la prise sur la partie d'anatomie rachidienne, est étroitement liée - mais non exclusivement - à sa forme au moins en partie complémentaire à la partie de l'anatomie rachidienne sur laquelle elle est destinée à venir se fixer, ou venir en prise. Par exemple, la forme ou la géométrie du moyen d'ancrage, en tout ou partie, sera adaptée lorsque la partie d'anatomie rachidienne consiste en une lame pédiculaire, lombaire ou transverse ;
- le crochet d'ancrage est amovible par rapport au corps de fixation par exemple par clipsage, collage et/ou vissage ;
- le corps de liaison, la tige et le crochet d'ancrage sont en une même matière, de préférence en titane ;
- le corps de fixation, ainsi que le crochet d'ancrage en tout ou partie, sont en matériau polymère ;
- la bande souple est une tresse en polymère ;
- le corps de fixation est de section transversale en forme de U ou sensiblement en forme de U, présentant une première branche, un fond et une seconde branche, les moyens de blocage réglable étant formés par une pièce de liaison reliant les extrémités en vis à vis des deux branches du U, les dites branches comprenant chacune un évidement en vis à vis situé du côté du fond du U, à savoir un premier évidement pour le passage d'une ou des extrémités libres de la bande et un deuxième évidement de l'autre côté pour le passage de la bande propre à former une boucle de fixation sur la vertèbre, la pièce de liaison étant formée par une vis de serrage munie d'un côté d'une tête de passage dans la première branche du U, comportant une partie agencée pour comprimer la tige au fur et à mesure de son vissage, et comprimer ainsi les deux extrémités de la bande souple contre la paroi du fond du U, et de l'autre côté d'une extrémité de vissage sur la seconde branche du U opposée. ;
- la paroi de fond du U est en forme de demi-cylindre de forme complémentaire à celle de la tige, terminé de part et d'autre par des rebords longitudinaux autorisant un clipsage de la tige dans le fond du U grâce à une légère déformation (inférieure à 1 mm) des branches.

L'invention concerne également un système de redressement d'une colonne vertébrale comprenant au moins deux dispositifs tels que décrits ci-dessus et au moins une tige.

Avantageusement, il comprend deux tiges et au moins quatre dispositifs tels que décrits ci-dessus.

Egalement avantageusement, le système comprend un moyen de mise en tension des bandes souples de chaque dispositif. De préférence, ce moyen de mise en tension est du type de ceux décrits dans le document FR 2 968 739, déposé par la demanderesse.

Le dispositif selon l'invention et tel que décrit ci-dessus peut être mis en place par une méthode de fixation, non revendiquée, d'une vertèbre sur une tige.

Elle propose aussi un procédé de fixation d'une vertèbre sur une tige par un corps de fixation muni d'un crochet et présentant une section transversale en forme de U ou sensiblement en forme de U agencé pour se fixer sur ladite tige par le biais d'une vis de liaison entre les branches du U dont l'une est munie d'un alésage fileté, ledit corps étant muni d'un évidement de passage d'une bande ou tresse souple de liaison d'une partie de ladite vertèbre avec le corps, caractérisé en ce que on introduit la tresse au travers du corps de fixation, on passe la tresse autour d'une lame de vertèbre, on repasse la tresse au travers du corps de fixation, de sorte qu'une boucle est formée autour de la lame de vertèbre, puis on insère le corps sur la tige, on introduit la vis dans le corps, la partie filetée de la vis étant juste mise en contact avec l'alésage fileté sans exercer de serrage pour permettre à la tresse de coulisser dans le corps, on solidarise les extrémités libres de la tresse que l'on met en tension pour rapprocher la tige de la colonne vertébrale, en comprimant les deux extrémités de la tresse contre la paroi du fond du U pour obtenir une première tension autorisant le rapprochement)
et, lorsque la lame de la vertèbre est à portée pour que le crochet puisse vernir s'encastrer sur la lame de vertèbre autour de laquelle est passée la boucle, on positionne la partie crochet au bon endroit en contact, en contrainte et/ou sensiblement en contrainte sur la lame de vertèbre, puis on visse définitivement ladite vis de serrage en position définitive de blocage de la tresse sur le corps de fixation.

Autrement et avantageusement, le corps de fixation étant de section transversale en forme de U tel que décrit ci-avant, (la pièce de liaison étant formée par une vis de serrage munie d'un côté d'une tête de passage dans une première branche du U, ladite tête comportant une partie tronconique dirigée vers l'autre branche), on introduit la tresse au travers du corps de fixation (dans le sens haut/bas), on passe la tresse autour d'une partie d'anatomie rachidienne (lame de vertèbre), on repasse la tresse au travers du corps de fixation (sens bas/haut) de sorte qu'une boucle est formée autour de la lame de vertèbre, puis on clipse le corps sur la tige, on introduit la vis dans le corps, la partie filetée étant juste mise en contact avec l'alésage fileté sans exercer de serrage pour permettre à la tresse de coulisser dans le corps, on solidarise les extrémités libres de la tresse que l'on met en tension pour rapprocher la tige de la colonne vertébrale, la tresse étant dès lors utilisée comme un guide le long duquel coulisse le corps, (alternativement on visse ladite vis de serrage de sorte qu'on comprime légèrement la tige par la partie tronconique au fur et à mesure de son vissage, et on comprime ainsi les deux extrémités de la bande souple contre la paroi du fond du U pour obtenir une première tension qui autorise néanmoins le rapprochement), lorsque la lame de la vertèbre est à portée pour que le crochet puisse vernir s'encastrer sur la partie d'anatomie rachidienne autour de laquelle est passée la boucle, on positionne la partie crochet au bon endroit, puis on visse définitivement ladite vis de serrage en position définitive de blocage de la bande sur le corps de fixation, les efforts étant alors repris en totalité et/ou en partie par le crochet.

Un éventuel desserrage de la bande devient alors sans effet et/ou conséquence.

Egalement avantageusement on arrête le vissage par blocage d'un épaulement de la tête de vis sur la face externe de la première branche du U.

Encore avantageusement on forme au moins deux boucles avec les bords de deux dispositifs, que l'on forme autour de deux parties d'anatomie, en passant leurs extrémités respectives dans au moins deux corps de fixation comme décrit ci-avant, on les clipse sur la tige puis on les met en légère compression par vissage, on rapproche la tige et on verrouille en position.

L'invention sera mieux comprise à la lecture de la description qui suit d'un mode de réalisation donné ci-après à titre d'exemple non limitatif.

La description se réfère aux dessins qui l'accompagnent dans lesquels :
La figure 1 est une vue en perspective du dispositif de fixation avec tige cylindrique, selon un mode de réalisation de l'invention.
La figure 2 est une vue de dessus montrant le dispositif de la figure 1 fixé sur une vertèbre.
La figure 3 est une vue en coupe axiale et partielle du dispositif de la figure 1 avec tige cylindrique.
La figure 4 est une vue de dessus du dispositif de la figure 1.
La figure 5 est une vue en perspective, en pièces détachées du dispositif de la figure 1.
Les figures 6A et 6C montrent schématiquement les principales étapes de mise en oeuvre de la méthode de fixation d'un système selon un mode de réalisation de l'invention, sur une grande scoliose.

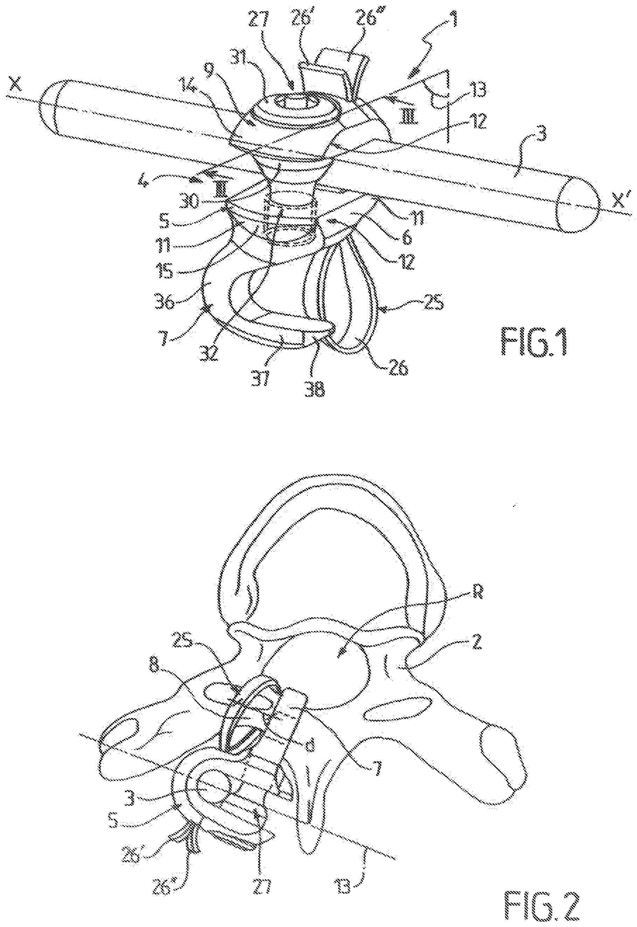

Les figures 1 à 5 montrent un dispositif 1 de fixation vertébrale, pour maintien d'une vertèbre rachidienne 2 sur une tige cylindrique 3.

Il comporte un élément 4 de fixation comprenant une pièce 5 d'un seul tenant, comprenant un corps 6 de fixation de section transversale sensiblement en forme de U et un crochet 7 d'ancrage rigide solidaire du corps de fixation, apte à venir se fixer (s'accrocher) sur la lame vertébrale 8 de la vertèbre.

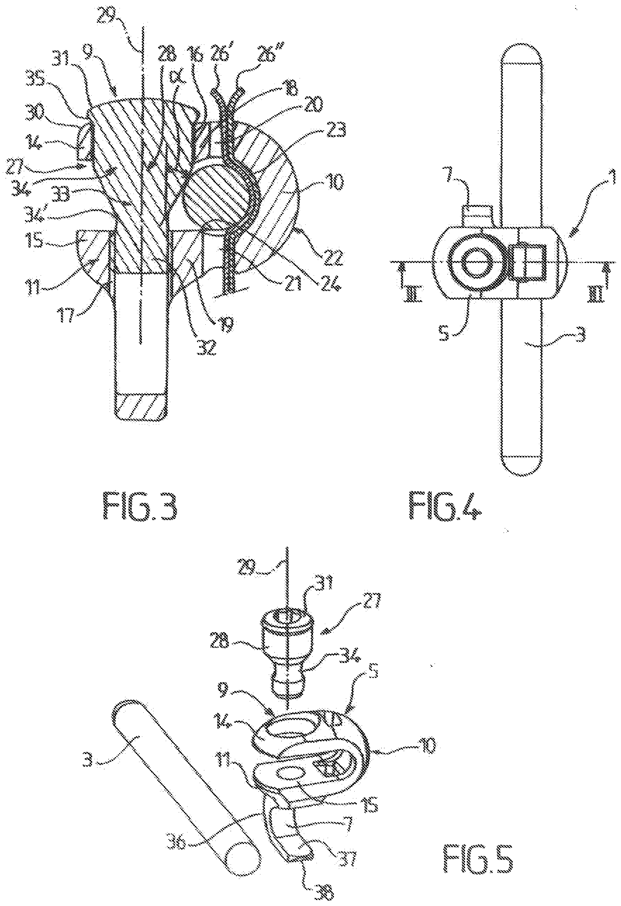

Le corps 6 (cf. figure 3) présente une première branche 9, un fond 10 et une seconde branche 11.

Les première et seconde branches 9, 11 du U sont constituées par deux plaquettes ou languettes 12, par exemple de 5 mm à 8 mm d'épaisseur de section transversale sensiblement rectangulaire ou trapézoïdale, comprenant chacune deux parties latérales symétriques par rapport à un plan P transversal 13 (voir figure 1).

Chaque plaquette comporte une portion d'extrémité 14, 15 percée chacune d'un alésage 16, 17 et reliée au fond 10 par une portion rectangulaire ou sensiblement rectangulaire 18, 19, terminée par un évidement 20, 21, les évidements 20, 21 étant situés en vis à vis l'un de l'autre du côté du fond 10.

Le fond 10 comporte quant à lui d'un côté une paroi externe 22 arrondie, de surface en partie cylindrique (ou en partie torique), et de l'autre côté une paroi interne 23 homothétique ou parallèle à la paroi externe 22, constituant le fond d'une gorge cylindrique de passage et de forme sensiblement complémentaire à celle de la tige 3.

Dans le mode de réalisation plus particulièrement décrit ici (cf. figure 3) la paroi 23 comprend en partie inférieure et/ou supérieure, un rebord longitudinal 24 dans le sens parallèle à la tige 3, formé par l'arête supérieure de la portion sensiblement parallélépipédique 19, qui va permettre l'enclipsage de la tige lorsqu'elle est bloquée par la pression comme on va le voir.

Le dispositif 1 de fixation comprend de plus une boucle 25 de fixation sur la partie d'anatomie rachidienne formée par la lame 8 en passant par le canal rachidien R, à proximité immédiate (distance d de quelques millimètres) du crochet 7 (voir figure 2).

La boucle est formée par une bande 26 souple en polymère tressé, par exemple en polyester de 1 à 3 mm d'épaisseur, de 6 mm de large et de 30 cm de long.

Plus précisément la boucle 25 est formée par le rapprochement des deux portions d'extrémité 26', 26" de la bande 26 souple et assure donc la liaison de la vertèbre 2 avec la pièce 5 de fixation.

A noter que les évidements 20 et 21 sont situés (pour leurs portions situées du côté du fond) en vis-à-vis l'un de l'autre et forment chacun une fente large, par exemple 5 à 10 fois plus large qu'une double épaisseur de bande 26, et ce pour faciliter son introduction lors de l'opération.

Le dispositif 1 comprend également des moyens 27 de blocage des deux portions d'extrémité 26', 26" de la bande 26 sur la paroi 23 du fond 10 du U, par la tige 3.

Plus précisément en référence aux figures 1, 3 et 5 les moyens 27 de blocage sont réglables et comprennent une vis 28 de liaison agencée pour relier les portions d'extrémité 14, 15 en vis-à-vis des deux branches 9 et 11 du U.

La vis 28 s'étend autour d'un axe longitudinal 29 et comprend une partie supérieure 30 cylindrique terminée d'un côté par une tête 31 pour le vissage et le blocage longitudinal de la vis par rapport à la première branche 9 et de l'autre côté par une portion tronconique 34 de blocage progressif de la tige lors du vissage (effet de coin).

La partie tronconique 34 s'étend par exemple sur une hauteur de la vis 28 comprise entre 1/6^{ème} et 1/3 de celle-ci et forme un angle par exemple de 15° avec l'axe 29.

La vis 28 comporte également une partie inférieure 32 cylindrique munie d'un pas de vis pour vissage dans l'alésage fileté 17 de la seconde branche 11 du U.

Dans le mode de réalisation décrit ici, la partie supérieure 30 et la partie inférieure 32 sont reliées entre elles par une partie intermédiaire 33 formant une gorge annulaire 34' par exemple de 0, 5 mm.

La tête 31 de la vis 28 de serrage comporte quant à elle un épaulement 35 propre à coopérer avec la face externe supérieure de la branche 9.

A nouveau en référence plus particulièrement à la figure 1, le crochet d'ancrage 7 s'étend, à partir du corps 6 de fixation, dans le même sens que la boucle 25 la tresse souple 26. Les éléments 25 et 7 sont ainsi situés du même côté du dispositif de fixation, en dessous du plan de la tige.

Ce crochet d'ancrage 7 est ici solidaire du corps 6 de fixation et s'étend à partir d'une portion extrêmale de la branche 11 du corps 6 de fixation. Plus précisément, dans le mode de réalisation plus particulièrement décrit ici, le crochet d'ancrage 7 s'étend à partir de la branche 11 du corps 6 de fixation en U, sur l'un des côtés, dit inférieur, de cette branche. Ainsi, le crochet d'ancrage 7 s'étend ou se situe dans le prolongement axial de la vis 28, entièrement mais décalé par rapport à l'évidement 21 autorisant le passage de la tresse souple 26, et donc par rapport au fond 23 contre lequel la tige 3 vient bloquer ladite bande.

Le crochet d'ancrage 7 consiste ici en un crochet présentant deux portions 36 et 37, à savoir une première portion proximale cylindrique (de section transversale en demi-cercle) et une deuxième portion distale allongée ou en forme de plaque (de section transversale linéaire) située dans un plan perpendiculaire à l'axe 29 de la vis. L'extrémité 38 du moyen d'ancrage 7, à l'extrémité libre de la deuxième portion, est par exemple biseautée.

Ainsi que cela a été présentée précédemment, la forme ou la géométrie du moyen d'ancrage 7 peut être variée mais elle doit permettre une prise aisée avec la partie 8 de l'anatomie rachidienne sur laquelle elle doit venir se fixer ou prendre appui.

Les figures 3 à 5 permettent de bien appréhender, selon des vues diverses, le mode de réalisation considéré ici pour illustrer l'invention à titre d'exemple schématique non limitatif.

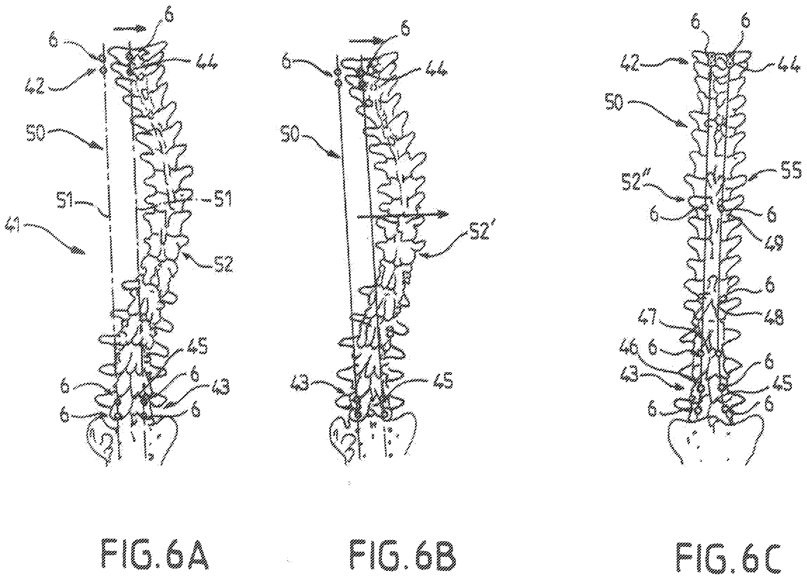

On a représenté sur les figures 6A à 6C une colonne vertébrale 40 avant et après réduction avec un système 41 selon l'invention (figuré schématiquement en trait mixte sur les figures).

Le montage complet, ou système de fixation, peut potentiellement être utilisé de l'occiput à la dernière vertèbre lombaire (L5).

Le système de fixation selon l'invention permet de connecter mécaniquement les tresses souples 26 des dispositifs décrits ci-dessus, ainsi que les crochets d'ancrage 7 avec les vertèbres 44 et 45, puis de compléter avec des vertèbres intermédiaires 46, 47, 48, 49.

On rappelle que la fixation du crochet d'ancrage 7 est une fixation libre, dans le sens où elle consiste en une simple prise sur la partie 8 de l'anatomie rachidienne, tandis que la fixation par la tresse souple 25 permet une immobilisation pérenne du corps 6 de fixation, et donc de la tige 3 (51) d'union, à une distance prédéterminée de la vertèbre 2, plus précisément de la partie 8 de l'anatomie rachidienne considérée.

On va maintenant décrire, en référence plus particulièrement aux figures 1, 2 et 6A à 6B, le fonctionnement et/ou la mise en oeuvre du dispositif 1 et du système 50 correspondant.

La méthode de fixation va permettre le blocage en position serrée des boucles 25 formée par les deux extrémités 26', 26" des bandes 26 souples à différents endroits sur les tiges 51, à l'aide des pièces 5 et des corps 6 de fixation, pour autoriser la fixation mécanique des boucles 25 autour des vertèbres 44, 45 etc concernées.

L'utilisation d'un tel système 50 est particulièrement indiquée dans le cadre d'une chirurgie du rachis du type grande scoliose, mettant en oeuvre les deux tiges parallèles 51 placées de part et d'autre de la colonne 52 du patient, dont les positions vont évaluer en 52', 52".

A noter également qu'un tel système et/ou dispositifs peut s'avérer très utile dans des applications dégénératives, traumatologiques ou tumorales, en renfort de vis pédiculaires implantées dans un os de mauvaise qualité.

Dans d'autres cas de pathologies dégénératives primaires, il peut être envisagé d'utiliser uniquement un tel dispositif en remplacement des vis pédiculaires. Dans cette optique et afin de garder une certaine souplesse à la portion de colonne vertébrale opérée on peut vouloir utiliser des tiges en polymère possédant un module d'élasticité proche de celui de l'os. Dans ce cas, il est nécessaire d'optimiser le contact avec la tige afin d'éviter un fluage pouvant être néfaste à la survie à long terme du système.

Plus précisément le chirurgien va procéder comme suit.

Après ouverture de la portion dorsale du patient (non représenté), pour accéder aux vertèbres à redresser, le chirurgien dispose les deux tiges 51.

Chaque corps 3 de fixation de dispositif 6, va ensuite être placé à la distance désirée de la vertèbre grâce à la bande/tresse souple 26, en mettant le moyen d'ancrage 7 en position de prise ultérieure.

Une première tresse est introduite par une extrémité dans le corps 3, puis passée en boucle autour de la vertèbre concernée, avant d'être réintroduite dans le corps 3.

La même opération est réalisée sur la ou les vertèbres d'à côté que l'on souhaite repositionner les unes par rapport aux autres, pour ici réparer la scoliose.

Le chirurgien clipse ensuite la ou les dispositifs avec tresse sur les tiges correspondantes, puis réalise ensuite la réduction avec les dispositifs 1 (en matériau biocompatible) comme suit.

Il est décrit ci-après uniquement l'opération avec un dispositif. Il va de soi que la pose et l'utilisation des autres dispositifs va être réalisée de façon similaire et progressivement équilibrée.

Dans l'exemple décrit, la vis 28 de fixation, la tige 3 et le corps 6 de liaison sont en titane.

Le chirurgien, qui a donc formé la boucle 25 de façon lâche autour de la lame 8 de la vertèbre 43, et passé les portions d'extrémité 26', 26" de la bande 26 dans les évidements 20 et 21 du corps 6, les fait donc ressortir de l'autre côté par rapport à la boucle.

Il vient ensuite encliquer le dispositif sur la tige 3 qui vient comprimer légèrement les deux portions d'extrémité 26', 26" de la bande contre le fond du U, ce qui lui permet un premier ajustage de la largeur de la boucle.

Il insère alors la vis 28 dans l'alésage de la première branche 9 supérieure jusqu'à venir coopérer avec l'alésage 17 de la seconde branche 11.

La boucle 25 est alors rapprochée de la tige 51 en mettant en tension les deux liens d'extrémités 26', 26" de façon connue en elle-même, par exemple en utilisant un dispositif de mise en tension du type décrit en référence au document FR 2 968 739, ce qui a pour résultat de rapprocher progressivement la vertèbre 44 de la tige 51 et de redresser la partie basse de la colonne, le frottement des portions d'extrémité contre le fond du U étant dosé.

Dans un autre mode de réalisation correspondant au cas d'une utilisation en dégénératif, il n'y a pas de phase de réduction proprement dite. Il s'agit simplement de mettre la tresse en tension alors que la tige est déjà correctement positionnée. Il n'y a donc qu'un rapprochement minime entre la vertèbre et la tige 3 jusqu'à ce que le crochet 7 puisse être introduit sur la lame 8.

Lorsque la position recherchée est obtenue, il y a alors introduction du crochet puis vissage progressif de la vis, ce qui vient pousser la partie conique de la vis sur la tige jusqu'à butée longitudinale de l'épaulement 7 de la tête 31 de la vis contre le bord supérieur de la branche 9.

L'opération est alors répétée avec les vertèbres supérieures ce qui permet le redressement progressif de la colonne jusqu'à arriver en phase finale (cf. figure 6C), à une colonne sécurisée, y compris en partie médiane par l'utilisation des dispositifs complémentaires 55 sur les vertèbres 46, 47, 48, 49.

Comme il va de soi et comme il résulte également de ce qui précède, la présente invention n'est pas limitée aux modes de réalisation plus particulièrement décrits. Elle en embrasse au contraire toutes les variantes et notamment celles où deux ou plusieurs tiges sont fixées à la suite ou de part et d'autre de la colonne vertébrale, celle où la vis et/ou le crochet sont de formes différentes.

## Revendications

1. Dispositif (1) de fixation vertébrale pour maintien d'une vertèbre rachidienne (2) sur une tige (3, 51), comportant
ladite tige s'étendant autour d'un axe,
un corps (6) de fixation sur la tige (3, 51) de section transversale en U ou sensiblement en forme de U présentant une première branche (9), un fond (10) et une seconde branche (11), chaque branche comportant un évidement (20, 21), les évidements étant situés en vis à vis l'un de l'autre du côté du fond (10) pour définir un passage sensiblement perpendiculaire à la tige,
une bande (26) souple de liaison d'une partie de ladite vertèbre avec le corps (6) de fixation, ladite bande formant une boucle (25) par rapprochement de ses deux portions d'extrémités (26', 26") insérées dans lesdits évidements en vis à vis du côté du fond, définissant un plan d'extension de la boucle perpendiculaire à l'axe de la tige en position de repos,
et des moyens (27) de blocage du corps sur la tige (3, 51) et de la bande (26) souple sur le corps (6) de fixation via la tige (3, 51),
**caractérisé en ce que** le corps est muni d'un crochet d'ancrage rigide,
**en ce que** ledit crochet est solidaire d'une des branches du U de l'autre côté du corps par rapport au fond, est parallèle à la tige, est situé entièrement à distance du passage de la bande défini par les évidements de l'autre côté du plan parallèle audit passage de la bande, défini par l'axe de la tige, par rapport au passage formé par les évidements et **en ce que** ledit crochet est agencé pour venir s'encastrer sur la partie de vertèbre à proximité immédiate de la bande (26) souple lorsque celle-ci est en position resserrée sur ladite partie de vertèbre.

2. Dispositif selon la revendication 1, **caractérisé en ce que** le crochet d'ancrage (7) présente une section transversale sensiblement en forme de L agencée pour s'insérer sur ladite partie de vertèbre.

3. Dispositif selon l'une quelconque des revendications 1 et 2, **caractérisé en ce que** le crochet d'ancrage (7) présente une section de forme au moins en partie complémentaire à la partie de l'anatomie rachidienne sur laquelle il est destiné à venir en prise, afin d'assurer une fixation amovible par complémentarité de formes.

4. Dispositif selon l'une quelconque des revendications précédentes, **caractérisé en ce que** le crochet d'ancrage (7) est amovible par rapport au corps (6) de fixation.

5. Dispositif selon l'une quelconque des revendications précédentes, **caractérisé en ce que** le corps (6) de liaison, la tige (3, 51) et le crochet d'ancrage (7) sont en titane.

6. Dispositif selon l'une quelconque des revendications précédentes, **caractérisé en ce que** le corps (6) de fixation et le crochet d'ancrage (7) en tout ou partie, sont en matériau polymère.

7. Dispositif selon l'une quelconque des revendications précédentes, **caractérisé en ce que** la bande (26) souple est une tresse (25) en polymère.

8. Dispositif selon l'une quelconque des revendications précédentes, **caractérisé en ce que** les moyens (27) de blocage réglable étant formés par une pièce de liaison reliant les extrémités en vis à vis des deux branches (9, 11) du U, ladite pièce de liaison est formée par une vis (28) de serrage munie d'un côté d'une tête de passage dans la première branche (9) du U, comportant une partie agencée pour comprimer la tige (3, 51) au fur et à mesure de son vissage, et comprimer ainsi les deux extrémités (26', 26") de la bande (26) souple contre la paroi (23) du fond (10) du U, et de l'autre côté d'une extrémité de vissage sur la seconde branche (11) du U opposée.

9. Dispositif selon l'une quelconque des revendications précédentes, **caractérisé en ce que** la paroi (23) de fond (10) du U est en forme de demi cylindre de forme complémentaire à celle de la tige (3, 51), terminé de part et d'autre par des rebords longitudinaux (24) autorisant un clipsage de la tige (3, 51) dans le fond (10) du U grâce à une légère déformation des branches (9, 11).

10. Système (50, 41) de redressement d'une colonne (52) vertébrale (40) comprenant au moins deux dispositifs selon l'une quelconque des revendications 1 à 9 et au moins une tige (3, 51).

## Patentansprüche

1. Wirbelfixiervorrichtung (1) zum Halten eines Wirbels (2) an einem Stift (3, 51), umfassend:
den sich um eine Achse erstreckenden Stift,
einen Körper (6) zur Befestigung an dem Stift (3, 51) mit einem U-förmigen oder im Wesentlichen U-förmigen Querschnitt mit einem ersten Schenkel (9), einen Boden (10) und einem zweiten Schenkel (11), wobei jeder Schenkel eine Ausnehmung (20, 21) aufweist, wobei die Ausnehmungen auf der Seite des Bodens (10) einander gegenüberliegen, um eine im Wesentlichen senkrecht zum Stift verlaufende Durchführung zu definieren, einen flexiblen Streifen (26) zum Verbinden eines Teils des Wirbels mit dem Befestigungskörper (6), wobei der Streifen eine Schleife (25) durch Annäherung seiner zwei Endabschnitte (26', 26") aneinander bildet, die in die bodenseitig einander gegenüberliegenden Ausnehmungen eingeschoben werden, wodurch eine Ebene zur Verlängerung der Schleife definiert wird, die senkrecht zur Achse des Stiftes in Ruhestellung verläuft, und
Mittel (27) zur Arretierung des Körpers an dem Stift (3, 51) und des flexiblen Streifens (26) an dem Körper (6) zur Befestigung durch den Stift (3, 51),
**dadurch gekennzeichnet,**
**dass** der Körper mit einem starren Verankerungshaken versehen ist,
**dass** der Haken auf der anderen Seite des Körpers in Bezug auf den Boden mit einem der Schenkel des U fest verbunden ist, parallel zu dem Stift verläuft, in Bezug auf die durch die Ausnehmungen gebildete Durchführung vollständig beabstandet zur Durchführung des Streifens angeordnet ist, die durch die Ausnehmungen auf der anderen Seite der parallel zur Streifendurchführung verlaufenden Ebene definiert ist, welche durch die Achse des Stiftes definiert ist, und
**dass** der Haken angeordnet ist, um auf den Wirbelabschnitt in unmittelbarer Nähe des flexiblen Streifens (26) aufgesteckt zu werden, wenn dieser sich in festgezogener Stellung auf dem Wirbelabschnitt befindet.

2. Vorrichtung nach Anspruch 1,
**dadurch gekennzeichnet,**
**dass** der Verankerungshaken (7) einen im Wesentlichen L-förmigen Querschnitt aufweist, der angeordnet ist, um auf den Wirbelabschnitt aufgesteckt zu werden.

3. Vorrichtung nach einem der Ansprüche 1 und 2,
**dadurch gekennzeichnet,**
**dass** der Verankerungshaken (7) einen mindestens teilweise komplementär zu dem Abschnitt der Wirbelsäulenanatomie ausgebildeten Querschnitt aufweist, mit dem er in Eingriff gelangen soll, um eine durch Formschlüssigkeit lösbare Befestigung sicherzustellen.

4. Vorrichtung nach einem beliebigen der vorhergehenden Ansprüche,
**dadurch gekennzeichnet,**
**dass** der Verankerungshaken (7) in Bezug auf den Befestigungskörper (6) lösbar ist.

5. Vorrichtung nach einem beliebigen der vorhergehenden Ansprüche,
**dadurch gekennzeichnet,**
**dass** der Verbindungskörper (6), der Stift (3, 51) und der Verankerungshaken (7) aus Titan sind.

6. Vorrichtung nach einem beliebigen der vorhergehenden Ansprüche,
**dadurch gekennzeichnet,**
**dass** der Befestigungskörper (6) und der Verankerungshaken (7) ganz oder teilweise aus einem Polymermaterial sind.

7. Vorrichtung nach einem beliebigen der vorhergehenden Ansprüche,
**dadurch gekennzeichnet,**
**dass** der flexible Streifen (26) ein Polymergeflecht (25) ist.

8. Vorrichtung nach einem beliebigen der vorhergehenden Ansprüche,
**dadurch gekennzeichnet,**
**dass** bei Bildung der Mittel (27) für eine regulierbare Arretierung durch ein Verbindungsstück, das die einander gegenüberliegenden zwei Schenkel (9, 11) des U verbindet, das Verbindungsstück durch eine Klemmschraube (28) gebildet ist, die auf einer Seite mit einem Kopf zur Durchführung durch den ersten Schenkel (9) des U versehen ist mit einem Abschnitt, der angeordnet ist, um den Stift (3, 51) mit fortschreitender Verschraubung zusammenzudrücken und somit um die zwei Enden (26', 26") des flexiblen Streifens (26) gegen die Wand (23) des Bodens (10) des U zu drücken, und auf der anderen Seite mit einem Ende für die Verschraubung an dem zweiten, entgegengesetzten Schenkel (11) des U versehen ist.

9. Vorrichtung nach einem beliebigen der vorhergehenden Ansprüche,
**dadurch gekennzeichnet,**
**dass** die Wand (23) des Bodens (10) des U die Form eines Halbzylinders aufweist, der komplementär zur Form des Stifts (3, 51) ausgebildet ist und beidseitig mit länglichen Rändern (24) endet, die durch eine leichte Verformung der Schenkel (9, 11) ein Aufclipsen auf den Stift (3, 51) auf dem Boden (10) des U ermöglicht.

10. System (50, 41) zum Aufrichten einer Säule (52) aus Wirbeln (40) mit mindestens zwei Vorrichtungen nach einem der Ansprüche 1 bis 9 und mindestens einem Stift (3, 51).

## Claims

1. Vertebral fixing device (1) for maintaining a spinal vertebra (2) on a rod (3, 51), comprising
said rod extending about an axis,
a body (6) for fixing to the rod (3, 51) of U-shaped or substantially U-shaped cross-section having a first arm (9), a base (10) and a second arm (11), each arm comprising a recess (20, 21), the recesses being situated opposite each other on the side of the base (10) in order to define a passage substantially perpendicular to the rod,
a flexible band (26) for connecting a part of said vertebra to the fixing body (6), said band forming a loop (25) by moving closer together its two end portions (26', 26") which are inserted in said recesses opposite on the side of the base, defining a loop extension plane perpendicular to the axis of the rod in the rest position,
and means (27) for locking the body to the rod (3, 51) and locking the flexible band (26) to the fixing body (6) via the rod (3, 51),
**characterised in that** the body is provided with a rigid anchoring hook,
**in that** said hook is rigidly connected to one of the arms of the U on the other side of the body from the base, is parallel to the rod, is situated completely remotely from the passage of the band defined by the recesses on the other side of the plane parallel to said passage of the band, defined by the axis of the rod, relative to the passage formed by the recesses
and **in that** said hook is arranged to fit over the vertebra part in the immediate proximity of the flexible band (26) when the latter is in the position tightened over said vertebra part.

2. Device according to claim 1, **characterised in that** the anchoring hook (7) has a substantially L-shaped cross-section arranged to be inserted on said vertebra part.

3. Device according to any one of claims 1 and 2, **characterised in that** the anchoring hook (7) has a cross-section of a shape at least partly complementary to the part of the spinal anatomy over which it is intended to become engaged, in order to ensure removable fixing by complementarity of shapes.

4. Device according to any one of the preceding claims, **characterised in that** the anchoring hook (7) is removable relative to the fixing body (6).

5. Device according to any one of the preceding claims, **characterised in that** the connecting body (6), the rod (3, 51) and the anchoring hook (7) are made of titanium.

6. Device according to any one of the preceding claims, **characterised in that** the fixing body (6) and the anchoring hook (7) are partly or completely made of polymeric material.

7. Device according to any one of the preceding claims, **characterised in that** the flexible band (26) is a plait (25) made of polymer.

8. Device according to any one of the preceding claims, **characterised in that** as the adjustable locking means (27) are formed by a connecting piece connecting the opposite ends of the two arms (9, 11) of the U, said connecting piece is formed by a clamping screw (28) provided on one side with a head for passage in the first arm (9) of the U, comprising a portion arranged to compress the rod (3, 51) as it is being screwed, and so compress the two ends (26', 26") of the flexible band (26) against the wall (23) of the base (10) of the U, and on the other side with an end for screwing onto the opposite second arm (11) of the U.

9. Device according to any one of the preceding claims, **characterised in that** the wall (23) of the base (10) of the U is semi-cylindrical with a shape complementary to that of the rod (3, 51), ending on either side in longitudinal edges (24) allowing the rod (3, 51) to be clipped on in the base (10) of the U owing to slight deformation of the arms (9, 11).

10. System (50, 41) for straightening a vertebral (40) column (52) comprising at least two devices according to any one of claims 1 to 9 and at least one rod (3, 51).
